# EUROPÄISCHE PATENTANMELDUNG

(11) **EP 2 692 367 A2**
(43) Veröffentlichungstag der Anmeldung: **05.02.2014**
(21) Anmeldenummer: 13175701.5
(22) Anmeldetag: 09.07.2013
(51) Int. Cl.: A61L 29/02, A61L 31/02

(54) **Medizinische Vorrichtung zur intrakorporalen Anwendung**

(30) Priorität: 31.07.2012 US 201261677490 P
(71) Anmelder: Biotronik AG, 8180 Bülach (CH)
(72) Erfinder: Blohm, Christian, 90419 Nürnberg (DE)
(74) Vertreter: Lindner-Vogt, Karin L.

(57) **Zusammenfassung**

Die Erfindung betrifft eine medizinische Vorrichtung zur intrakorporalen Anwendung. Die erfindungsgemäße medizinische Vorrichtung zur intrakorporalen Anwendung zeichnet sich dadurch aus, dass sie zumindest teilweise aus Aerographit geformt ist.

## Beschreibung

Die vorliegende Erfindung betrifft eine medizinische Vorrichtung zur intrakorporalen Anwendung.

Der Einsatz von Implantaten oder medizinischen Geräten zur intrakorporalen Diagnostik oder Behandlung stellt einen sehr wesentlichen Aspekt moderner Medizintechnik dar. Für diese Zwecke entwickelte medizinische Vorrichtungen müssen vielfältigsten Anforderungen genügen. Abgesehen von der eigentlichen Zweckbestimmung der Vorrichtungen ist beispielsweise die Materialwahl bei Implantaten für die dauerhafte Verträglichkeit von erheblicher Bedeutung.

Unter Implantat wird allgemein jede medizinische Vorrichtung aus einem oder mehreren Werkstoffen verstanden, die absichtlich in den Körper eingebracht wird und entweder teilweise oder ganz von einer Epitheloberfläche bedeckt ist. Implantate lassen sich hinsichtlich der Verwendungsdauer in Temporär- und Permanentimplantate untergliedern. Temporärimplantate verbleiben für einen befristeten Zeitraum im Körper. Permanentimplantate sind für den dauerhaften Verbleib im Körper vorgesehen. Bei Implantaten kann ferner zwischen Prothesen und künstlichen Organe unterschieden werden. Eine Prothese ist eine medizinische Vorrichtung, die Gliedmaßen, Organe oder Gewebe des Körpers ersetzt, während unter einem künstlichen Organ eine medizinische Vorrichtung verstanden wird, die teilweise oder ganz die Funktion eines Körperorgans ersetzt. Unter die genannten Definitionen fallen beispielsweise Implantate wie orthopädische oder osteosynthetische Implantate, Herzschrittmacher und Defibrillatoren und vaskuläre Implantate.

Insbesondere die Implantation von Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmen-stents bekannt, die vorrangig zur Abdichtung des Aneuryrismas dienen.

Stents besitzen eine Umfangswandung von ausreichender Tragkraft, um das verengte Gefäß im gewünschten Maße offen zu halten, und einen rohrförmigen Grundkörper durch den der Blutfluss ungehindert weiterläuft. Die Umfangswandung wird in der Regel von einer gitterartigen Tragstruktur gebildet, die es erlaubt, den Stent in einem komprimierten Zustand mit kleinem Außendurchmesser bis zur zu behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist. Alternativ besitzen Form-Gedächtnis-Materialien wie Nitinol die Fähigkeit zur Selbstexpansion, wenn eine Rückstellkraft wegfällt, die das Implantat auf einem kleinen Durchmesser hält. Die Rückstellkraft wird in der Regel durch einen Schutzschlauch auf das Material ausgeübt.

In allen von der Umgebung aus zugänglichen Bereichen der medizinischen Vorrichtung bestehen besondere Anforderungen an den verwendeten Werkstoff. Grundvoraussetzungen für den Einsatz eines Werkstoffes, der bei bestimmungsgemäßen Zweck mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Werkstoffs ist weiterhin abhängig vom zeitlichen Ablauf der Reaktion des Biosystems, in das implantiert wird oder das zumindest temporär mit dem Werkstoff in Kontakt tritt. So treten relativ kurzfristig Reizungen und Entzündungen auf, die zu Gewebeveränderungen führen können. Biologische Systeme reagieren demnach in Abhängigkeit von den Eigenschaften des Werkstoffs in verschiedener Weise. Entsprechend der Reaktion des Biosystems können die Werkstoffe in bioaktive, bioinerte und degradierbare/resorbierbare Werkstoffe unterteilt werden.

Werkstoffe, die für die medizinische Anwendung geeignet sind, umfassen Polymere, metallische Werkstoffe und keramische Materialien (zum Beispiel als Beschichtung). Biokompatible Metalle und Metalllegierungen für Permanentimplantate oder andere medizinische Vorrichtungen zur intrakorporalen Anwendung beinhalten beispielsweise rostfreie Stähle, Kobaltlegierungen, Reintitan und Titanlegierungen und Goldlegierungen. Im Bereich biokorrodierbarer Implantate, insbesondere Stents, wird der Einsatz von Magnesium oder Reineisen sowie biokorrodierbaren Basislegierungen der Elemente Magnesium, Eisen, Zink, Molybdän und Wolfram vorgeschlagen.

Abgesehen von Implantaten, werden medizinische Vorrichtungen auch zur Diagnose oder Behandlung im Körper eingesetzt. Beispielsweise ist die perkutane transluminale Angioplastie (PTA) ein Verfahren zur Erweiterung oder Wiedereröffnung von verengten oder verschlossenen Blutgefäßen mittels Ballondilatation. Dazu wird insbesondere ein Ballonkatheter über einen Führungsdraht und Führungskatheter in die Stenose platziert und mit Druck aufgeblasen, wodurch die Stenose beseitigt wird. Medikamentenbeschichtete/freisetzende Ballonkatheter sind eine Weiterentwicklung herkömmlichen Ballonkatheter. Die Ballonoberfläche ist hierbei mit einem Medikament (zum Beispiel dem Zytostatikum Paclitaxel) beschichtet, das an der Stelle der Gefäßverengung aufgetragen wird. Das Medikament soll ein gefäßverengendes Überwuchern der erweiterten Stelle verhindern.

Die erfindungsgemäße medizinische Vorrichtung zur intrakorporalen Anwendung zeichnet sich nun dadurch aus, dass sie zumindest teilweise aus Aerographit geformt ist. Mit anderen Worten, Teile (zum Beispiel ein Grundkörper der Vorrichtung) oder die gesamte medizinische Vorrichtung enthält Aerographit oder besteht aus Aerographit.

Die medizinische Vorrichtung kann insbesondere ein Implantat oder ein Ballonkatheter sein. Aerographit findet demnach erstmalig als Werkstoff in der Medizintechnik Verwendung. Vorzugsweise weist die medizinische Vorrichtung eine Beschichtung auf, die Aerographit enthält oder aus Aerographit besteht.

Aerographit ist ein neues, ultraleichtes Kohlenstoffmaterial. Eine detaillierte Beschreibung des Materials sowie ein Weg zu dessen Herstellung ist in M. Mecklenburg, A. Schuchardt, Y. K. Mishra, S. Kaps, R. Adelung, A. Lotnyk, L. Kienle, K. Schulte "Aerographite: Ultra Lightweight, Flexible Nanowall, Carbon Microtube Material with Outstanding Mechanical Performance" Advanced Materials 2012, 24, 3486-3490, beschrieben.

Aerographit hat eine mikroporöse Struktur, gute mechanische Eigenschaften und ist biokompatibel, wodurch es sich hervorragend als Beschichtung von medizinischen Geräten zur intrakorporalen Anwendung eignet, insbesondere für Implantaten oder Ballonkatheter. Vorzugsweise ist das Aerographit mit einem Wirkstoff (Arzneimittel) beladen. Über seine mikroporöse Struktur kann Aerographit dabei mit beliebigen Wirkstoffen beladen werden. Durch seine große Oberfläche und elektrische Leitfähigkeit ist Aerographit ebenfalls als Material für oder zur Beschichtung von Elektroden, insbesondere Elektroden von Herzschrittmachern oder Defibrillatoren, geeignet.

Aerographit wird unter Verwendung eines Zinkoxid Opfertemplates erzeugt. Die aus Zinkoxid bestehende Struktur (das Opfertemplate) wird zu diesem Zweck mit einem kohlenstoffhaltigen Gas, beispielsweise Toluol, begast. Dadurch entsteht eine Kohlenstoffnanostruktur um das Opfertemplate. Danach wird das Zinkoxid mittels Wasserstoff reduziert und ausgegast. Die entstandene Struktur wird mit Argon gespült und abgekühlt. Über Prozessparameter, wie Gasfluss (kohlenstoffhaltiges Gas, Wasserstoff), Temperatur (Begasung, Reduktion, Abkühlung) und die Dauer der unterschiedlichen Prozessschritte, können die Eigenschaften des Materials weiter variiert werden. So können insbesondere Aerographite unterschiedlicher Dichte erzeugt werden.

Nach einer bevorzugten Ausführungsform enthält das Aerographit Zinkoxid. Das zinkoxidhaltige Aerographit lässt sich beispielsweise durch nur unvollständige Reduktion und Ausgasung des als Opfertemplate verwendeten Zinkoxids herstellen. Zinkoxid besitzt eine entzündungshemmende Wirkung und unterstützt somit den Heilungsprozess nach intrakorporaler Behandlung.

Die medizinische Vorrichtung ist insbesondere ein Implantat. Implantate im Sinne der Erfindung sind über ein chirurgisches Verfahren in den Körper eingebrachte Vorrichtungen und umfassen Befestigungselemente für Knochen, beispielsweise Schrauben, Platten oder Nägel, chirurgisches Nahtmaterial, Darmklammern, Gefäßclips, Prothesen im Bereich des Hart- und Weichgewebes und Elektroden. Das Implantat besteht zumindest in Teilen aus Aerographit. Insbesondere weist das Implantat eine Beschichtung auf, die Aerographit enthält oder aus Aerographit besteht. Vorzugsweise ist das Implantat ein Stent oder eine implantierbare Elektrode, insbesondere für Schrittmacher oder Defibrillatoren.

Gemäß einem Aspekt der Erfindung wird demnach ein medizinisches Implantat, insbesondere ein Stent, mit einem Aerographit beschichtet. Dabei wird bevorzugt ein Opfertemplate (wie vorangehend beschrieben) um einen Stent mit geeigneter Schmelztemperatur (beispielsweise L605 mit einer Schmelztemperatur von 1.300-1.400°C) gesintert. Wie oben beschrieben wird ein kohlenstoffhaltiges Gas injiziert und das Opfertemplate reduktiv entfernt. Dabei kann der Herstellungsprozess beispielsweise über Parameter, wie Form und Dichte des Opfertemplates, Temperatur sowie Gasfluss, gesteuert werden. Das derart entstandene Implantat, insbesondere der Stent, befindet sich nach diesem Prozess in einer Schaum-/Schwammhülle aus Aerographit, die mit beliebigen, insbesondere hydrophoben und medizinisch wirksamen Substanzen (Wirkstoffen) beladen werden kann. Im Falle eines Stents kann die Beladung dabei vor oder nach dem als "Crimpen" bekannten Prozess, d. h. dem Aufbringen des Stents auf einen Ballon eines Ballonkatheters zum Einbringen des Stents in ein Körpergefäß, erfolgen.

Die derart entstandene dichte, aber poröse Hülle aus Aerographit ist auf Grund ihrer hydrophoben Eigenschaften geeignet, Rupturen (Risse) in Körpergefäßen zu verschließen. Dieser Effekt kann noch durch die Beladung der Aerographitbeschichtung mit einem geeigneten Wirkstoff verstärkt werden. Optional kann das Implantat, insbesondere der Stent, mit der Aerographitbeschichtung auch ohne Beladung mit Wirkstoff an den Anwender, insbesondere den Arzt, geliefert werden. Die Beladung kann dann direkt am Ort der Behandlung vom Anwender durch ein einfaches Tauchverfahren (sogenanntes Dipcoating) vorgenommen werden. Dadurch kann der Anwender selbst Wirkstoff und Dosierung auswählen.

Gemäß einem anderen Aspekt der Erfindung wird das Implantat, insbesondere der Stent, selbst aus Aerographit hergestellt. Dabei wird vorzugsweise das Opfertemplate in die Form des Implantats gebracht und der Gasaustauschprozess (Begasung, Reduktion) wie vorangehend beschrieben durchgeführt.

Insbesondere wird ein Stent aus Aerographit wie vorangehend beschrieben hergestellt. Ein derartiger Stent ist hinreichend mechanisch stabil, um ein Körpergefäß zu stützen. Dies gilt insbesondere bei der Behandlung einer Stenose in einem Körpergefäß, wo das Körpergefäß mittels Ballon aufgeweitet und ein Stent eingebracht wird, um die Aufweitung des Körpergefäßes aufrecht zu erhalten. Aerographit lässt sich stark komprimieren und nimmt anschließend wieder die ursprüngliche Form ein. Daher kann ein derartiger Stent aus Aerographit selbstexpandierend ausgeführt werden.

Bei einem Stent oder einem anderen Implantat aus Aerographit ist keine zusätzliche Beschichtung zur Beladung mit einem medizinischen Wirkstoff notwendig. Aerographit ist, wie vorangehend beschrieben, hochporös, so dass das Implantat, insbesondere der Stent, selbst direkt mit dem Wirkstoff beladen werden kann. Die Beladung kann dabei analog zu der vorangehend beschriebenen Beladung einer Beschichtung aus Aerographit erfolgen, einschließlich des Beladens durch den Anwender.

Der Stent aus Aerographit gemäß dieser Ausgestaltung der Erfindung kann dabei nahezu jedes im Stand der Technik bekannte Design aufweisen. Insbesondere kann der Stent aufgrund der porösen Struktur des Aerographites als einfache Röhre ausgeformt werden.

Die poröse Struktur des Aerographites fördert dabei optimal das Einwachsen des Implantats, insbesondere des Stents, in das umgebende Körpergewebe, insbesondere in das umgebende Körpergefäß.

Gemäße einem weiteren Aspekt der Erfindung ist das Implantat eine Elektrode, insbesondere eine Elektrode eines Herzschrittmachers oder Defibrillators. Die Elektrode kann dazu wie vorangehend geschildert mit Aerographit beschichtet oder aus Aerographit hergestellt werden. Eine Elektrode nach diesem Aspekt der Erfindung wächst schneller in das umgebende Körpergewebe ein und weist eine verbesserte Impulsübertragung durch die größere, fraktale Oberfläche des Aerographits auf.

Gemäß einem weiteren Aspekt der Erfindung ist die medizinische Vorrichtung ein Ballonkatheter, der beispielsweise zur Behandlung einer Stenose in einem Körpergefäß durch Aufweiten des Körpergefäßes dient. Insbesondere wird ein Ballon des Ballonkatheters mit Aerographit beschichtet, d. h. der Ballon weist eine Beschichtung auf, die Aerographit enthält oder aus Aerographit besteht. Dazu wird bevorzugt ein Röhrchen aus Zinkoxid mit einem Innendurchmesser, der ungefähr dem Durchmesser des Ballons in seinem gefalteten Zustand entspricht, als Opfertemplate verwendet. Unter dem gefalteten Zustand eines Ballons wird der Zustand des Ballons verstanden, den der Ballon bei Einführung/Einbringen in das Körpergefäß hat. Aus dem Opfertemplateröhrchen wird analog zu den vorangehende beschriebenen Verfahren ein Aerographitröhrchen erzeugt. Dieses Aerographitröhrchen wird anschließend auf den Ballon in seinem gefalteten Zustand gezogen und in geeigneter Weise (beispielsweise durch Verkleben) befestigt. Die derart entstandene Aerographitbeschichtung des Ballons kann dann wie vorangehend geschildert mit einem geeigneten Wirkstoff in der flüssigen Phase beladen werden. Alternativ kann der Ballon von einer Hülse umgeben sein, die Aerographit enthält oder aus Aerographit besteht und in geeigneter Weise auf dem Ballonkatheter fixiert ist.

Bei der Behandlung der Stenose im Körpergefäß durch Aufweiten/Expandieren des Ballons wird das Aerographit durch Ballon einerseits und Körpergefäß andererseits komprimiert, wodurch im Falle einer Wirkstoffbeladung der Wirkstoff aus dem Aerographit freigesetzt wird. Auf diese Weise kann der Wirkstoff direkt und gezielt bei der Behandlung einer Stenose in das Körpergefäß eingebracht werden. Nach dem Entspannen/Dilatation des Ballons geht das Aerographit wieder in seine ursprüngliche Form zurück und kann mit dem Ballonkatheter aus dem Körpergefäß entfernt werden.

Die vorliegende Erfindung weist insbesondere folgende Vorteile auf: Aerographit kann nachträglich mit beliebigen medizinisch wirksamen Substanz beladen werden, was eine sehr flexible Anpassung der medizinischen Vorrichtung an die jeweiligen Erfordernisse vor Ort ermöglicht. Beispielsweise kann der Anwender Dosierung und Wirkstoffauswahl variieren. Implantate, insbesondere Stents, können einfacher produziert und in ihren Eigenschaften verbessert werden.

Ein weiterer Aspekt der Erfindung liegt in der neuen Verwendung von Aerographit in der Medizintechnik, speziell in medizinischen Vorrichtungen zur intrakorporalen Anwendung.

## Patentansprüche

1. Medizinische Vorrichtung zur intrakorporalen Anwendung, **dadurch gekennzeichnet, dass** die medizinische Vorrichtung zumindest teilweise aus Aerographit geformt ist.

2. Medizinisches Vorrichtung nach Anspruch 1, bei dem die medizinische Vorrichtung eine Beschichtung aufweist, die Aerographit enthält oder aus Aerographit besteht.

3. Medizinisches Vorrichtung nach einem der vorhergehenden Ansprüche, bei dem die medizinische Vorrichtung ein Implantat ist.

4. Medizinisches Vorrichtung nach Anspruch 3, bei dem das Implantat ein Stent ist.

5. Medizinisches Vorrichtung nach Anspruch 3, bei dem das Implantat eine Elektrode, insbesondere eines Herzschrittmachers oder Defibrillators, ist.

6. Medizinisches Vorrichtung nach Anspruch 1, bei dem die medizinische Vorrichtung ein Ballonkatheter ist.

7. Medizinisches Vorrichtung nach Anspruch 6, bei dem ein Ballon des Ballonkatheters eine Beschichtung aufweist oder von einer Hülse umgeben ist, die Aerographit enthält oder aus Aerographit besteht.

8. Medizinisches Vorrichtung nach einem der vorhergehenden Ansprüche, bei dem das Aerographit mit einem Wirkstoff beladen ist.

9. Medizinisches Vorrichtung nach einem der vorhergehenden Ansprüche, bei dem das das Aerographit Zinkoxid enthält.

10. Verwendung von Aerographit zur Herstellung einer medizinischen Vorrichtung zur intrakorporalen Anwendung.
